# EUROPÄISCHE PATENTSCHRIFT

(11) **EP 2 802 368 B1**
(45) Veröffentlichungstag und Bekanntmachung des Hinweises auf die Patenterteilung: **18.07.2018**
(21) Anmeldenummer: 13701360.3
(22) Anmeldetag: 10.01.2013
(51) Int. Cl.: A61B 50/20, A61M 5/14, A61B 90/57

(54) **HALTERUNG FÜR EINE ABLAGE**
HOLDER FOR A TRAY
SYSTEME DE FIXATION POUR UN PLATEAU

(30) Priorität: 12.01.2012 DE 102012000410; 12.01.2012 US 201261585841 P
(43) Veröffentlichungstag der Anmeldung: 19.11.2014
(73) Patentinhaber: Fresenius Medical Care Deutschland GmbH, 61352 Bad Homburg (DE)
(72) Erfinder: BREHM, Winfried, 97461 Hofheim (DE)
(74) Vertreter: Schön, Andrea
(86) Internationale Anmeldenummer: PCT/EP2013/000045
(87) Internationale Veröffentlichungsnummer: WO 2013/104536

(56) Entgegenhaltungen:
- US-A- 5 114 023
- US-A1- 2011 147 550
- US-B1- 6 409 131
- US-B2- 7 967 137

## Beschreibung

Die Erfindung betrifft eine Halterung nach Anspruch 1 für die Anbringung eines Gegenstandes, z.B. eines Ablagetabletts an der Infusionsstange eines medizinischen Gerätes, wie z.B. einer Dialysemaschine. Damit wird dem Pflege- und Servicepersonal eine Fläche für die Ablage von Gebrauchsgegenständen an dem Behandlungsplatz zur Verfügung gestellt.

### Stand der Technik

Im Stand der Technik sind Vorrichtungen bekannt, die die Anbringung und Aufbewahrung unterschiedlichster Gegenstände direkt an einer Infusionsstange ermöglichen.

In der US 2007/0267551 ist eine Halterung für eine Infusionsstange beschrieben, die zur Aufnahme der persönlichen Gegenstände eines Patienten dient, damit er diese zusammen mit der Infusionsstange ständig mit sich führen kann. Diese Halterung wird mit einer Schraubklemme an der Infusionsstange befestigt. Solche Schraubklemmen bestehen aus mehreren Teilen und sind durch ihren Aufbau nur sehr schwer zu reinigen. Im medizinischen Umfeld spielt die Hygiene eine große Rolle und um eine einfache Reinigung und Desinfektion zu ermöglichen, werden glatte Flächen bevorzugt.

In der US 2005/0016044 erfolgt die Befestigung von Gegenständen, z.B. von Rahmen, an der Infusionsstange mithilfe einer biegsamen Metallkabels, das um die Stange gewickelt wird. Die Montage und auch die Deinstallation sind eher umständliche Arbeiten.

In der US 2009/0294604 sind Befestigungsmittel beschrieben, die mit einer Hand angebracht werden können, und deren Fixierung an der Stange über Reibung erfolgt. Ein Nachteil dieser Vorrichtung ist, dass sie ohne Belastung leicht verrutschen kann. Deshalb müssen in der Infusionsstange spezielle Klemmbacken vorgesehen sein, um die Halterungen in der gewünschten Position zu halten, wenn bei fehlender Gewichtsbelastung die Reibung nicht zur Fixierung genügt.

In der US 7,967,137 ist eine Ablage beschrieben, die an eine Halterung an einer Infusionsstange eingeklemmt werden kann. Diese Halterung ist mittels einer Schraubklemme an der Infusionsstange befestigt.

Ferner offenbart die US 6,409,131 B1 eine Halterung gemäß des Oberbegriffs von Anspruch 1.

### Problemstellung

Bei der Behandlung von Patienten, insbesondere der Dialysebehandlung, benötigen die Ärzte und Pfleger direkt am Behandlungsplatz unterschiedliche Gebrauchsmaterialien, wie z.B. Desinfektionsmittel, Pflaster, Zugangskanülen usw. Üblicherweise werden diese z. B. auf der Oberfläche des Dialysegerätes aufbewahrt. Bei einigen Dialysegeräten ist der Bildschirm, der als Anzeigemittel und auch als Eingabemittel dienen kann, nicht in das Gerätegehäuse integriert. Der Bildschirm ist z.B. über einen beweglichen Tragarm an der Geräteoberfläche befestigt. Diese Befestigung kann auch zur Verankerung einer Infusionsstange dienen. Damit kann der Anwender den Bildschirm je nach örtlichen Gegebenheiten in unterschiedliche Positionen bringen, die ein Ablesen der Informationen und eventuell Eingabe von Behandlungsdaten bequem ermöglicht. Mit dieser Konstruktion eignet sich allerdings die Oberfläche des Gerätegehäuses nicht mehr als Ablagefläche.

Zudem werden einige Gebrauchsmaterialien an jedem Behandlungsplatz vom Pflegepersonal benötigt. Sie müssen dann entweder an jedem Behandlungsplatz gelagert werden, was wegen des herrschenden Platzmangels nachteilig ist, oder alternativ auf einem Tablett vom Pflegepersonal von Behandlungsplatz zu Behandlungsplatz transportiert werden.

Der Erfindung liegt die Aufgabe zugrunde, dem Arzt oder dem Pflegepersonal eine Ablagefläche direkt am Behandlungsgerät zur Verfügung zu stellen.

Nach der Lehre der Erfindung wird diese Aufgabe durch eine Halterung nach den Merkmalen des Anspruches 1 und der Verwendung der Halterung gemäß Anspruch 7 sowie einer Dialysemaschine nach Anspruch 8 gelöst. Vorteilhafte Ausgestaltungen sind Grundlage der Unteransprüche.

### Zusammenfassung der Erfindung

Die Erfindung betrifft eine Halterung für die lösbare Anbringung einer Ablage, die an einer Infusionsstange eines medizinischen Gerätes, insbesondere einer Dialysemaschine, befestigt werden kann. Die Halterung besteht aus einer Fläche, an die am äußeren Rand einstückig wenigstens ein Träger angebracht ist. An diesem Träger kann lösbar eine Ablage, wie z.B. ein Tablett, befestigt werden. Weiterhin am äußeren Rand der Fläche ist senkrecht zu der Fläche ein Stützmittel ausgeführt, das die Form eines Zylinder- oder Kegelausschnitts aufweist. Die Fläche weist zudem eine zentrale Ausnehmung auf, die zur Aufnahme der Infusionsstange dient. Zur Führung der Infusionsstange in diese zentrale Ausnehmung ist eine Ausklinkung vom äußeren Rand der Fläche zu der zentralen Ausnehmung vorgesehen. Erfindungsgemäß ist die Fläche weitgehend kreisförmig gestaltet. Um die Halterung an der Infusionsstange anzubringen, muss die Infusionsstange in die zentrale Ausnehmung eingeführt werden. Die dazu vorgesehene Ausklinkung ist gebogen ausgeführt. Durch die Biegung wird ein Haken ausgebildet, der eine besonders sichere Befestigung bildet.
Der Bildschirm des medizinischen Gerätes kann an einem Tragarm befestigt sein. Dabei kann sich zwischen Bildschirm und Tragarm ein bewegliches Gelenk befinden. Der Tragarm selbst kann auch zweiteilig ausgeführt sein, wobei die beiden Teile durch ein Gelenk verbunden sind. Der Tragarm kann mit einer Befestigung, die z.B. zylinder- oder kegelförmig ausgeführt sein kann, an der Geräteoberfläche angebracht sein. Der Tragarm kann um die Befestigung drehbar sein, so dass der Anwender den Bildschirm zu unterschiedlichen Seiten des Gerätes orientieren kann. Weitere Freiheitsgrade bezüglich der Orientierung des Bildschirms erhält der Anwender durch die beiden Gelenke in dem Tragarm.
Der Bildschirm kann zur Anzeige von Informationen für den Anwender dienen oder als Touchscreen auch zur Eingabe von Behandlungsdaten oder Behandlungsparametern verwendet werden. Die Halterung kann in einer bevorzugten Ausführungsform so gestaltet sein, dass die weitgehend kreisförmige Fläche und das Stützmittel formschlüssig mit einer Befestigung für den Tragarm des Bildschirms an der Geräteoberfläche zusammenpassen. Der Zylinder- oder Kreisausschnitt des Stützmittels kann sich vorzugsweise über einen Winkel zwischen 110 und 180° erstrecken.

In einer bevorzugten Ausführungsform besteht der an der Fläche angebrachte Träger für die Ablage aus mindestens zwei Haltemitteln. Dabei kann ein oberes Haltemittel als Haken ausgeführt sein, in den der Rand der Ablage eingehängt werden kann. Ein unteres Haltemittel kann als Auflagefläche ausgeführt sein, die die Ablage von unten stützt.

Die Halterung ist um die Infusionsstange drehbar. Dabei kann die Halterung so ausgeführt sein, dass sie sich bei Drehung des Tragarmes für den Bildschirm um die Infusionsstange aufgrund des Reibungswiderstandes zwischen der Befestigung des Tragarmes an der Geräteoberfläche und der Halterung mit dem gleichen Winkelbetrag dreht. Das Ablagetablett wird also parallel um den Bildschirm um die Infusionsstange gedreht. Die Position von Bildschirm und Ablagetablett relativ zueinander bleibt gleich. Durch manuelle Fixierung des Tragarmes, des Bildschirmes, der Halterung oder des Ablagetabletts können Tragarm oder Halterung auch unabhängig voneinander gedreht werden. Damit kann die Position von Bildschirm und Ablagetablett relativ zueinander verändert werden.

Durch den vertikalen Rand des Stützmittels wird ein Anschlag für den Tragarm gebildet wird, über den hinaus eine Rotation nicht möglich ist. Damit werden Zusammenstöße von Bildschirm und Ablagetablett weitgehend vermieden.

Durch die gebogen ausgeführte Ausklinkung vom Rand der Fläche zur zentralen Ausnehmung ist die Halterung durch Einhaken an der Infusionsstange zu befestigen.

Die Halterung ist bevorzugt aus Kunststoff, wie z.B. PET oder ABS, gefertigt. Sie kann in einer alternativen Ausführungsform allerdings auch aus Metall bestehen.

Zur Vermeidung von Beschädigungen durch Abrieb kann die Halterung an dem Stützmittel einen Gleitstreifen aufweisen.

Die zentrale Ausnehmung kann mit einer Verstärkungsrippe versehen sein, um die Spannung zu reduzieren.

Die Erfindung betrifft weiterhin die Verwendung der Halterung nach Anspruch 1 zur Befestigung einer Ablagefläche an der Infusionsstange eines medizinischen Gerätes, insbesondere einer Dialysemaschine.

Die Erfindung betrifft weiterhin eine Dialysemaschine mit einer Befestigung für einen Tragarm mit einem Bildschirm und einer Infusionsstange, an der eine Halterung nach Anspruch 1 mit einer Ablage und befestigt sind.

Weitere Einzelheiten und Vorteile der Erfindung werden anhand der in den Zeichnungen dargestellten Ausführungsbeispiele näher beschrieben. Sie zeigen:
Figur 1: Perspektivische Ansicht einer erfindungsgemäßen Halterung mit Ablage und eines Bildschirmes an einer Infusionsstange eines medizinischen Gerätes
Figur 2: Draufsicht der Halterung mit kreisförmiger Fläche und Träger
Figur 3: Querschnitt der Halterung
Figur 4: Seitenansicht der Halterung

### Beschreibung

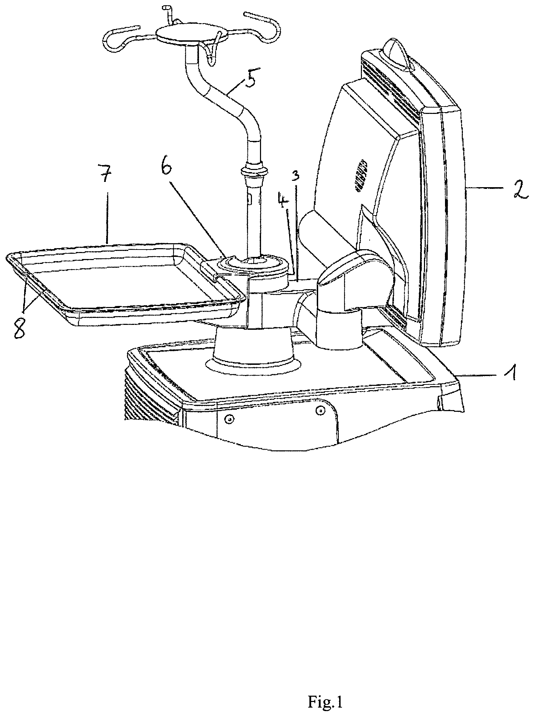

In der Abbildung 1 wird von schräg hinten eine Dialysemaschine 1 mit einer Infusionsstange 5 auf der Gehäuseoberfläche gezeigt. Die Infusionsstange 5 ist an einem Befestigungsmittel 4 für einen Tragarm 3 mit einem Bildschirm 2 angebracht. Das Befestigungsmittel 4 kann um die Mittelachse gedreht werden. Eine erfindungsgemäße Halterung 6 ist über dem Befestigungsmittel 4 an der Infusionsstange 5 eingehakt. An der Halterung 6 ist ein Ablagetablett 7 befestigt. Dieses besteht aus einer rechteckigen Fläche mit einem weitgehend senkrecht darauf stehenden Rand. Der Rand weist dabei an wenigstens einer Seite eine Aussparung 8 auf, die die seitliche Verschiebung des Ablagetabletts 7 in der Halterung 6 begrenzt. Die in Abbildung 1 gezeigte Ausführungsform weist die Aussparung 8 an zwei gegenüberliegenden Seiten auf, so dass eine Aussparung in die Halterung eingreift.

Die Halterung 6 und das Befestigungsmittel 4 für den Tragarm 3 passen form-schlüssig aufeinander. Der Reibungswiderstand zwischen Halterung 6 und Befestigungsmittel 4 bewirkt, dass wenn der Anwender den Bildschirm 2 mit dem Tragarm 3 um die Infusionsstange 5 dreht, die Halterung mitgedreht wird. Wird der Bildschirm 2 oder das Ablagetablett 7 fixiert, so können beide auch unabhängig voneinander um die Infusionsstange gedreht werden.

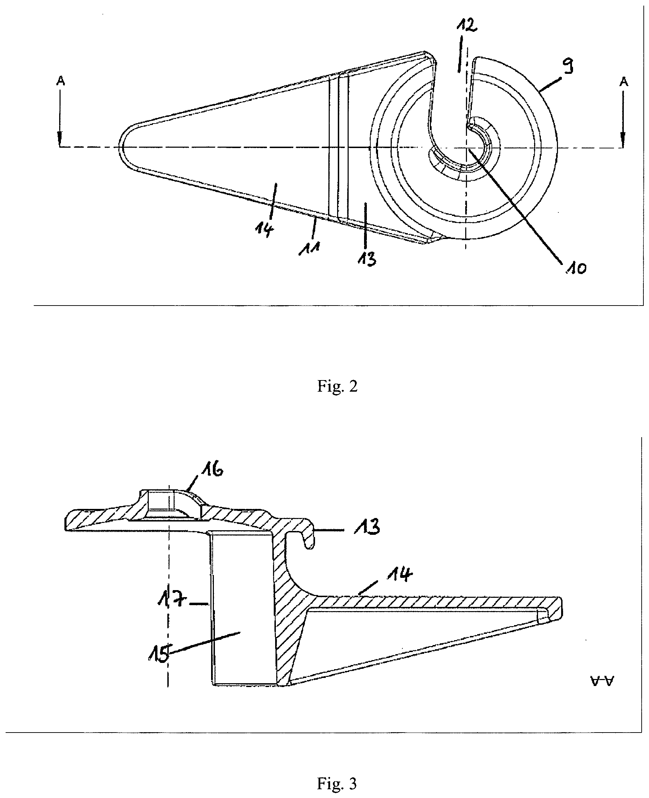

In der Abbildung 2 wird die Halterung 6 in der Draufsicht gezeigt. Sie weist eine weitgehend kreisförmige Fläche 9 mit einer zentralen Ausnehmung 10 auf. Vom äußeren Rand der Fläche 9 führt eine gebogene Ausklinkung 12 zu dieser zentralen Ausnehmung 10. Durch Führung der Infusionsstange 5 durch diese gebogenen Ausklinkung 12 zu der zentralen Ausnehmung 10 kann die Halterung in die Infusionsstange eingehakt werden. Dies kann ohne Zuhilfenahme von Werkzeugen und mit einer einzigen Hand erfolgen.

Die Symmetrieachse der Ausklinkung 12 und die Symmetrieachse des Trägers 11 stehen dabei ungefähr senkrecht aufeinander. So wird gewährleistet, dass bei einer Gewichtsbelastung der Halterung 6 und des Ablagetabletts 7 die Infusionsstange nicht aus der Halterung 6 herausrutschen kann, da die Hauptbelastung in der Symmetrieachse des Trägers 11 liegt. Durch die Biegung der Ausklinkung wird verhindert, dass auch in dem Fall, in dem das Tablett einseitig belastet wird, die Halterung nicht durch Kippen von der Infusionsstange gelöst werden kann.

An die Fläche 9 angeschlossen ist der Träger 11. Er besteht aus einem oberen Haltemittel 13, das als nach unten greifender Haken ausgebildet ist, und aus einem unteren Haltemittel 14, das hier als keilförmige Auflagefläche ausgebildet ist. Es sind allerdings auch andere Formen denkbar, die eine stabile Befestigung des Ablagetabletts bewirken.

Abbildung 3 zeigt einen Querschnitt der Halterung mit der Fläche 9 und weitgehend senkrecht zur Fläche 9 ein Stützmittel 15, das die Form eines Zylinder- oder Kegelausschnitts aufweist. Das Stützmittel 15 ist so ausgeführt, dass es mit der Fläche 9 zusammen weitgehend formschlüssig mit dem Befestigungsmittel 4 des Tragarmes 3 zusammenpasst. Die vertikale Abgrenzung des Stützmittels 15 bildet dabei einen Anschlag 17 für den Tragarm 3. Kollisionen zwischen Ablagetablett 7 und Bildschirm 2 werden bei Drehung des Ablagetabletts 7 oder des Bildschirms 2 um die Infusionsstange 5 weitgehend vermieden. An der Fläche 9 und dem Stützmittel 15 einstückig ausgebildet sind die beiden Haltemittel 13 und 14 des Trägers 11. Haltemittel 13 hat die Form eines nach unten gerichteten Hakens. Haltemittel 14 bildet eine waagerechte Auflagefläche. Durch leichtes Kippen kann das Ablagetabletts 7 in die Halterung eingeklemmt bzw. von ihr entfernt werden. Dies kann z.B. auch mit einem belegten Ablagetablett erfolgen.

Die Fläche 9, die als Auflagefläche auf das Befestigungsmittel 4 dient, weist hier weiterhin eine um die zentrale Ausnehmung führende Verstärkungsrippe 16 auf, die der Verformung der Halterung aufgrund von Spannungen bei Belastung des Ablagetabletts 7 entgegenwirkt.

Die Halterung 6 bestehend aus Fläche 9, Stützmittel 15 und Träger 11 mit den Haltemitteln 13 und 14, ist einstückig ausgeführt. Sie kann z.B. aus Kunststoff bestehen und durch Spritzguss hergestellt werden. Das Ablagetablett 7 ist lösbar mit der Halterung 6 zu verbinden. Die Halterung 6 ist lösbar an der Infusionsstange 5 anzubringen. Die Befestigung und Entfernung von Halterung 6 und Ablagetablett 7 sind durch die Form der Halterung sehr einfach und ohne Zuhilfenahme von Werkzeugen möglich. Die Dialysemaschine kann nachträglich mit dieser Halterung ausgerüstet werden. Eine Halterung kann je nach Bedarf leicht von einer Dialysemaschine zu einer anderen gewechselt werden. Da das Ablagetablett sehr leicht anzubringen und zu entfernen ist, kann der Pfleger z.B. auch ein Tablett mit seinen Bedarfsmaterialen ausrüsten und von einem zum nächsten Behandlungsplatz mit sich führen und dort an der Infusionsstange anbringen, sofern diese mit einer Halterung 6 versehen ist.

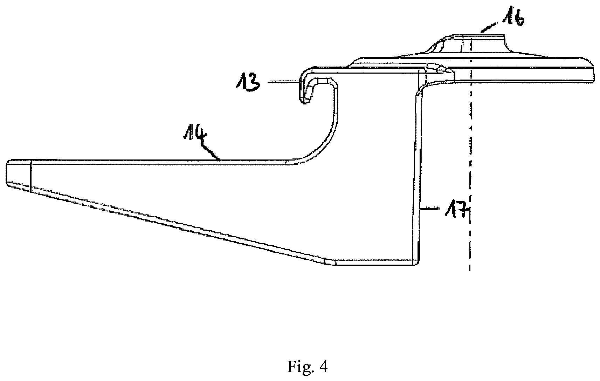

Abbildung 4 zeigt eine Seitenansicht der Halterung 6.

## Patentansprüche

1. Halterung (6) für die lösbare Anbringung einer Ablage (7) an einer Infusionsstange (5) eines medizinischen Gerätes (1), wobei die Halterung (6) aus einer weitgehend kreisförmigen Fläche (9) besteht und wobei am äußeren Rand der Fläche (9) einstückig wenigstens ein Träger (11) angebracht ist,
an den lösbar eine Ablage (7), befestigt werden kann, weiterhin am äußeren Rand ist weitgehend senkrecht zu der Fläche (9) wenigstens ein Stützmittel (15) in Form eines Zylinder- oder Kegelausschnitts ausgebildet, die Fläche (9) weist zudem eine zentrale Ausnehmung (10) auf, wobei vom äußeren Rand der Fläche (9) zu der zentralen Ausnehmung (10) eine Ausklinkung (12) vorgesehen ist, die zur Führung der Infusionsstange (5) in die zentrale Ausnehmung (10) dient
**dadurch gekennzeichnet, dass** die Ausklinkung (12) in Draufsicht gebogen ausgeführt ist.

2. Halterung (6) nach Anspruch 1 **dadurch gekennzeichnet, dass** sich der Zylinder- oder Kreisausschnitt des Stützmittels (15) sich über einen Winkel zwischen 110 und 180° erstreckt.

3. Halterung (6) nach einem der vorhergehenden Ansprüche **dadurch gekennzeichnet, dass** der Träger (11) aus mindestens zwei Haltemitteln (13,14) besteht, wobei ein oberes Haltemittel (13) als Haken und ein unteres Haltemittel (14) als Auflagefläche für die Ablage (7) ausgeführt sind.

4. Halterung (6) nach einem der vorhergehenden Ansprüche **dadurch gekennzeichnet, dass** die Halterung (6) um die Infusionsstange (5) drehbar ist.

5. Halterung (6) nach einem der vorhergehenden Ansprüche **dadurch gekennzeichnet, dass** die Halterung (6) an der Infusionsstange (5) über die Ausklinkung (12) einhakbar ist.

6. Halterung (6) nach einem der vorhergehenden Ansprüche **dadurch gekennzeichnet, dass** sie aus Kunststoff besteht.

7. Verwendung der Halterung (6) nach einem der Ansprüche 1 bis 6 zur Bereitstellung einer Ablagefläche (7) an der Infusionsstange (5) eines medizinischen Gerätes (1), insbesondere einer Dialysemaschine (1).

8. Dialysemaschine (1) mit einer Befestigung (4) mit einem Tragarm (3), mit einem Bildschirm (2) und mit einer Infusionsstange (5), **gekennzeichnet dadurch, dass** an der Infusionsstange eine Halterung (6) für die lösbare Anbringung einer Ablage (7) nach einem der Ansprüche 1 bis 6 angebracht ist.

9. Dialysemaschine nach Anspruch 8 **dadurch gekennzeichnet, dass** die weitgehend kreisförmige Fläche (9) und das Stützmittel (15) formschlüssig mit der Befestigung (4) für den Tragarm (3) für einen Bildschirm (2) an der Geräteoberfläche zusammenpassen.

10. Dialysemaschine (1) nach Anspruch 8 oder 9, **dadurch gekennzeichnet, dass** die Halterung (6) bei Drehung des Tragarmes (3) um die Befestigung (4) an der Geräteoberfläche aufgrund des Reibungswiderstandes zwischen der Befestigung (4) des Tragarmes (3) und der Halterung (6) mit dem gleichen Winkelbetrag gedreht wird.

11. Dialysemaschine (1) nach einem der Ansprüche 8 bis 10 **dadurch gekennzeichnet, dass** durch manuelle Fixierung von Tragarm (3) oder Halterung (6) beide auch unabhängig voneinander um die Infusionsstange (5) gedreht werden können.

12. Dialysemaschine (1) nach einem der Ansprüche 8 bis 11 **dadurch gekennzeichnet, dass** durch das Stützmittel (15) ein Anschlag für den Tragarm (3) gebildet wird, über den hinaus eine Rotation nicht möglich ist.

13. Dialysemaschine (1) nach einem der Ansprüche 8 bis 12 **dadurch gekennzeichnet, dass** das Stützmittel (15) an der Kontaktfläche zur Befestigung (4) des Tragarms (3) einen Gleitstreifen aufweist, die Beschädigungen der Befestigung des Tragarms (3) aufgrund von Abrieb verhindert.

## Claims

1. A holder (6) for releasable attachment of a tray (7) to an infusion rod (5) of a medical device (1), wherein the holder (6) consists of a largely circular area (9)
and wherein at least one support (11) is mounted in one piece on the outer edge of the area (9), such that a tray (7) can be attached releasably to said support; in addition, at least one supporting means (15) in the form of a cylindrical segment or a conical segment is formed on the outer edge where it is largely perpendicular to the area (9), the area (9) also having a central recess (10), wherein a notch (12), which serves to guide the infusion rod (5) into the central recess (10), is provided from the outer edge of the area (9) to the central recess (10), **characterized in that** the notch (12) is embodied with a curve as seen in a view from above.

2. The holder (6) according to claim 1, **characterized in that** the cylindrical or circular segment of the supporting means (15) extends over an angle between 110° and 180°.

3. The holder (6) according to any one of the preceding claims, **characterized in that** the support (11) consists of at least two holding means (13, 14), wherein an upper holding means (13) is embodied as a hook, and a lower holding means (14) is embodied as a support area for the tray (7).

4. The holder (6) according to any one of the preceding claims, **characterized in that** the holder (6) can be rotated about the infusion rod (5).

5. The holder (6) according to any one of the preceding claims, **characterized in that** the holder (6) can be hooked onto the infusion rod (5) by means of the notch (12) .

6. The holder (6) according to any one of the preceding claims, **characterized in that** it is made of plastic.

7. Use of the holder (6) according to any one of claims 1 to 6 for providing a support area (7) on the infusion rod (5) of a medical device (1), in particular a dialysis machine (1).

8. A dialysis machine (1) having a fastening (4) with a supporting arm (3) with a display screen (2) and with an infusion rod (5), **characterized in that** a holder (6) for releasable attachment of a tray (7) according to any one of claims 1 to 6 is mounted on the infusion rod.

9. The dialysis machine according to claim 8, **characterized in that** the largely circular area (9) and the supporting means (15) are fitted together in a form-fitting manner of the fastening (4) for the supporting arm (3) for the display screen (2) on the surface of the device.

10. The dialysis machine (1) according to claim 8 or 9, **characterized in that** the holder (6) is rotated about the fastening (4) on the device surface, with the same size of the angle based on the frictional resistance between the fastening (4) of the supporting arm (3) and the holder (6), in the rotation of the supporting arm (3) about the fastening (4).

11. The dialysis machine (1) according to any one of claims 8 to 10, **characterized in that** by manual fixation of the supporting arm (3) or the holder (6) the two can also be rotated independently of one another about the infusion rod (5).

12. The dialysis machine (1) according to any one of claims 8 to 11, **characterized in that** a stop for the supporting arm (3) is formed by means of the supporting means (15), so that rotation beyond this stop is impossible.

13. The dialysis machine (1) according to any one of claims 8 to 12, **characterized in that** the supporting means (15) have a sliding strip on the contact face with the fastening (4) of the supporting arm (3), preventing damage to the fastening of the supporting arm (3) due to abrasion.

## Revendications

1. Support (6) pour l'installation amovible d'une tablette (7) au niveau d'une barre de perfusion (5) d'un appareil médical (1), le support (6) consistant en une surface en grande partie circulaire (9) et étant installé au bord extérieur de la surface (9), au moins un élément porteur (11) en une pièce auquel une tablette (7) peut être fixée de manière amovible, étant en outre réalisé au bord extérieur, en grande partie perpendiculairement à la surface (9), au moins un moyen d'appui (15) sous forme d'une découpe cylindrique ou conique, la surface (9) présentant un autre évidement central (10), étant prévue, du bord extérieur de la surface (9) jusqu'à l'évidement central (10), une encoche (12) qui sert à guider la barre de perfusion (5) dans l'évidement central, **caractérisé en ce que** l'encoche (12), vue du dessus, a une conformation courbée.

2. Support (6) selon la revendication 1, **caractérisé en ce que** la découpe cylindrique ou circulaire du moyen d'appui (15) s'étend sur un angle de 110 à 180°.

3. Support (6) selon une des revendications précédentes, **caractérisé en ce que** l'élément porteur (11) est composé d'au moins deux moyens de retenue (13,14), un moyen de retenue supérieur (13) se présentant sous forme d'un crochet et un moyen de retenue inférieur (14) sous la forme d'une surface d'appui pour la tablette (7).

4. Support (6) selon une des revendications précédentes, **caractérisé en ce que** le support (6) peut tourner autour de la barre de perfusion (5).

5. Support (6) selon une des revendications précédentes, **caractérisé en ce que** le support (6) peut être accroché à la barre de perfusion (5) par l'encoche (12) .

6. Support (6) selon une des revendications précédentes, **caractérisé en ce qu'**il est composé de matière plastique.

7. Utilisation du support (6) selon une des revendications 1 à 6 pour créer une surface de dépôt (7) au niveau de la barre de perfusion (5) d'un appareil médical (1), en particulier d'une machine de dialyse (1).

8. Machine de dialyse (1) comportant une fixation (4) doté d'un bras porteur (3), d'un écran (2) et d'une barre de perfusion (5), **caractérisée en ce que**, au niveau de la barre d'infusion, est installé un support (6) pour l'installation amovible d'une tablette (7) selon une des revendications 1 à 6.

9. Machine de dialyse (1) selon la revendication 8, **caractérisée en ce que** la surface en grande partie circulaire (9) et le moyen d'appui (15) coïncident en correspondance géométrique avec la fixation (4) pour le bras porteur (3) d'un écran (2) à la surface de l'appareil.

10. Machine de dialyse (1) selon la revendication 8 ou 9, **caractérisée en ce que** le support (6), en cas de rotation du bras porteur (3) autour de la fixation (4) au niveau de la surface de l'appareil, en raison de la résistance de frottement entre la fixation (4) du bras porteur (3) et le support (6), est tourné avec la même amplitude angulaire.

11. Machine de dialyse (1) selon une des revendications 8 à 10, **caractérisée en ce que**, par une fixation manuelle du bras porteur (3) ou du support (6), tous deux peuvent aussi être tournés indépendamment l'un de l'autre autour de la barre de perfusion (5).

12. Machine de dialyse (1) selon une des revendications 8 à 11, **caractérisée en ce qu'**est formée par le moyen d'appui (15) une butée pour le bras porteur (3), butée au-delà de laquelle une rotation n'est pas possible.

13. Machine de dialyse (1) selon une des revendications 8 à 12, **caractérisée en ce que** le moyen d'appui (15), au niveau de la surface de contact avec la fixation (4) du bras porteur (3), présente une bande de glissement qui empêche les détériorations de la fixation du bras porteur (3) dues au frottement.
